# EUROPEAN PATENT SPECIFICATION

(11) **EP 2 914 307 B1**
(45) Date of publication and mention of the grant of the patent: **12.12.2018**
(21) Application number: 13808854.7
(22) Date of filing: 25.06.2013
(51) Int. Cl.: A61B 17/30, A61L 31/02, A61L 31/10, A61L 31/16, A61B 17/12

(54) **SYSTEMS AND METHODS FOR FABRICATING SPIRAL COILS WITH ATOMIZED BIOACTIVE COATINGS**
SYSTEME UND VERFAHREN ZUR HERSTELLUNG SPIRALFÖRMIGER SPULEN MIT ZERSTÄUBTEN BIOAKTIVEN BESCHICHTUNGEN
SYSTÈMES ET PROCÉDÉS DE FABRICATION D'ENROULEMENTS EN SPIRALE À REVÊTEMENTS BIOACTIFS ATOMISÉS

(30) Priority: 25.06.2012 WO PCT/US2012/044049; 20.08.2012 US 201261691244 P
(43) Date of publication of application: 09.09.2015
(73) Proprietor: The Regents of the University of California, Oakland, CA 94607 (US)
(72) Inventor: WU, Benjamin M., Los Angeles, California 90095-1600 (US); SUWARNASARN, Arnold, Los Angeles, California 90095-1600 (US); VINUELA, Fernando, Los Angeles, California 90095-7437 (US); YUKI, Ichiro, Los Angeles, California 90095-7437 (US)
(74) Representative: Farrington, Graham
(86) International application number: PCT/US2013/047713
(87) International publication number: WO 2014/004579

(56) References cited:
- WO-A1-2008/045475
- WO-A2-2004/096310
- WO-A2-2004/098671
- US-A1- 2004 138 695
- US-A1- 2008 124 452
- US-A1- 2008 171 129

## Description

### NOTICE OF MATERIAL SUBJECT TO COPYRIGHT PROTECTION

A portion of the material in this patent document is subject to copyright protection under the copyright laws of the United States and of other countries. The owner of the copyright rights has no objection to the facsimile reproduction by anyone of the patent document or the patent disclosure, as it appears in the United States Patent and Trademark Office publicly available file or records, but otherwise reserves all copyright rights whatsoever.

### BACKGROUND

### 1. Field of the Invention

This presentation relates generally to a bioactive coating on a medical device and methods of making and using the same, and more particularly to an intracranial aneurysm coil having a polymer coating on selected surfaces of the coil.

### 2. Description of Related Art

Subarachnoid hemorrhage from intracranial aneurysm rupture remains a devastating disease. Endovascular occlusion of ruptured and unruptured intracranial aneurysms using Guglielmi Detachable Coil (GDC) technology has gained worldwide acceptance as a less-invasive treatment alternative to standard microsurgical clipping. However, critical evaluation of the long-term anatomical results of aneurysms treated with metal coils shows three limitations. First, compaction and aneurysm recanalization can occur. This technical limitation is more often seen in small aneurysms with wide necks and in large or giant aneurysms. Second, the standard platinum metal coil is relatively biologically inert. Research relating to methods of enhancing the biological activity of metal coils highlights the increased interest in finding innovative solutions to overcome these present biological limitations of the conventional metal coil system.

Polymeric coatings carrying a bioactive agent have been used to impart bioactivity to implantable devices (e.g., stents). However, when a polymeric coating is formed on a spiral coil, often times, the grooves of the spiral coil are coated along with the outer surface of the coil, causing the mechanical flexibility to be compromised, which is undesirable. Further, for a spiral coil to be spatially compatible with a vascular lumen in the brain, sometimes it is important to limit the diameter of a coil to a certain size since it is constrained by the inner diameter of the microcatheter used for placing the coil.

WO 2008/045475 discloses a precision spraying device having an improved atomizing end for reproducibly forming droplets from small amounts of liquid with improved operational stability and spray pattern quality as well as a method of manufacturing. The document further provides a method for reproducibly coating substrates using such spraying device.

The present invention addresses the foregoing limitations of spiral intracranial aneurysm coils.

### BRIEF SUMMARY

The invention is described in the enclosed claims. Embodiments in this presentation comprise systems and methods for coating of spiral intracranial aneurysm coils, e.g., a Guglielmi Detachable Coil (GDC), such that only selected surfaces along the spiral coil are coated with a polymer via an atomized polymer deposition process. The resulting device is detachable aneurysm coil system which preserves the mechanical properties, flexibility, low friction, etc. to allow delivery, and low coil-coil entanglement to allow re-positioning of the coil, and delivers specific agents to promote wound healing.

The coated polymer coating may be bioactive, or may release a bioactive agent, or it may be of a type that reacts with the local environment to provide bulking function. Unlike liquid embolic agents, such as Onyx, which cannot be controlled in the context of placement or three dimensional shape, the coating material and processes of the present invention can be selected to remain in a solid state with defined shape and dimension for precise and accurate placement within the target.

These embodiments are compatible with existing coils (e.g. GDC's), which are already familiar to practitioners in the field. These embodiments preserve the mechanical handling of existing coils, while adding a bioactive agent. To preserve the flexibility and deliver specific agents to promote wound healing, the coating process of the present invention minimizes and confines the location of the polymer coating layer such that it does not impede the coils flexibility. By leaving the grooves between each coil segment uncoated, the coating preserves the mechanical flexibility of the coil.

The polymer coating of embodiments of this presentation provides two primary functions: to promote wound healing through intrinsic polymer properties, and to provide a potential vehicle for drug delivery. In addition, by employing post-processing techniques, the polymeric surface is improved to limit friction and wear of the coated surface.

Further aspects of embodiments of this presentation will be brought out in the following portions of the specification, wherein the detailed description is for the purpose of fully disclosing preferred embodiments without placing limitations thereon. Only embodiments covered by the scope of the claims form part of the invention.

### BRIEF DESCRIPTION OF THE SEVERAL VIEWS OF THE DRAWING(S)

This presentation will be more fully understood by reference to the following drawings which are for illustrative purposes only:
FIG. 1 is a side view of a nozzle performing atomized deposition in accordance with the present invention.
FIG. 2A is a side view of a GDC in a resting state, as compared to droplet size of the deposited coating.
FIG. 2B is a side view of a GDC of FIG. 2A in a stretched state.
FIG. 2C is a side view of a GDC of FIG. 2B with use of a mandrel
FIG. 3A is a cross-sectional view of an uncoated GDC.
FIG. 3B is a cross-sectional view of the GDC of FIG. 3A coated with a bioactive polymer.
FIG. 3C is a cross-sectional view of the GDC of FIG. 3B coated with a lubricant.
FIG. 4A and FIG. 4B show images of uncoated and coated GDC's respectively.
FIG. 5 is a flow diagram of a coating process in accordance with embodiments of this presentation.
FIG. 6 is a schematic diagram of a deposition system in accordance with embodiments of this presentation.

### DETAILED DESCRIPTION

In a preferred embodiment, system and methods are directed to atomized polymer deposition directly onto the surface of a Guglielmi Detachable Coil (GDC).

The polymer or combination of polymers is first solvated into a non-viscous solution. The GDC is elongated, along the coil axis, to create a linear target. The systems and methods employ atomization techniques which can be separated into two key components: droplet breakup, and the influence of solvent evaporation. In order to achieve discrete spiral coating along the coil, precise control of the atomized polymer particles is important. In general, discrete spiral coating can be achieved if atomized particles are smaller than the gap between adjacent coil segments

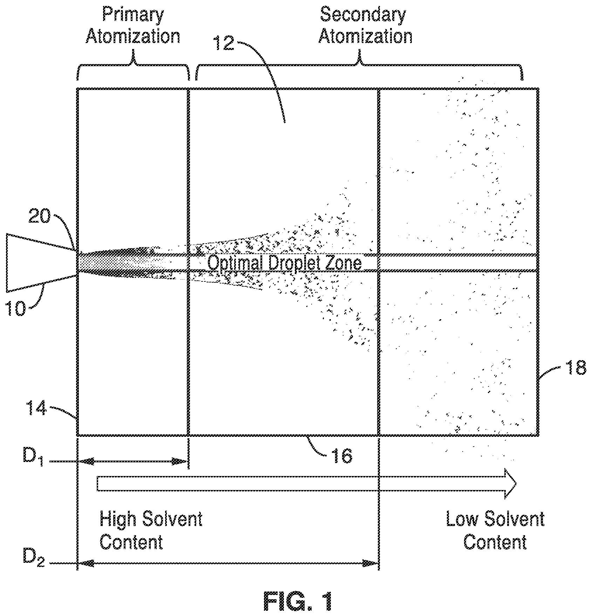

FIG. 1 is a side view of a nozzle 10 and corresponding atomized deposition droplet breakdown 12 in accordance with this presentation.

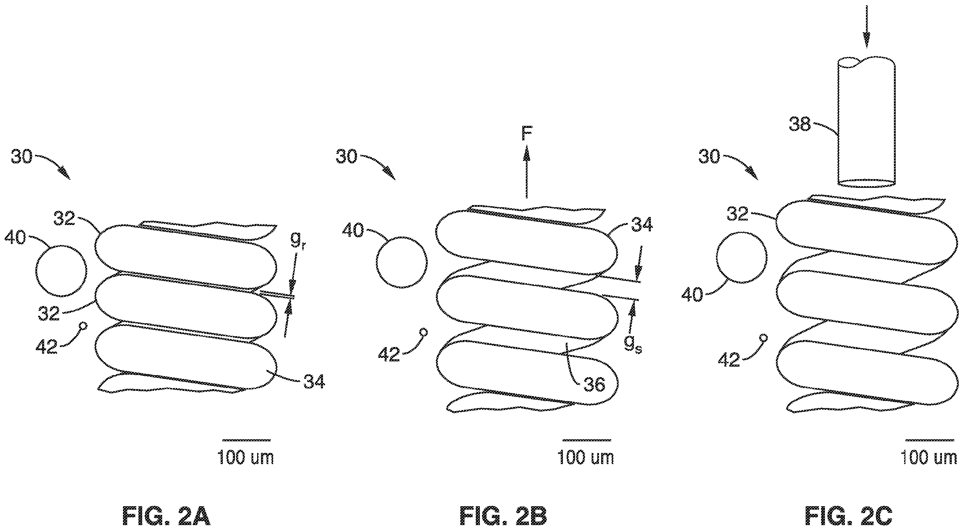

Droplet breakdown will be discussed first with reference to FIG. 1. Initially, atomized particles exiting distal end of nozzle 20 undergo primary atomization at region 14 that extends distance D₁ from the nozzle exit 20. The solution flow causes solution to exit the atomization nozzle 10, and dispensing air pressure causes initial breakup. In the primary atomization region 14, droplets are considered to be "immature," having a high solvent content and generally comprising large droplets (e.g. droplet 40 shown in FIG. 2A).

After primary atomization, secondary atomization takes place beyond distance D1 from the nozzle exit 20. The secondary atomization region comprises two sub-regions: 1) an optimal droplet zone 16 spanning from D₁ to distance D₂ from the nozzle exit 20, and 2) low-solvent region 18 extending beyond distance D₂ from the nozzle exit 20. In region 16, immature droplets break down further into smaller droplets (e.g. droplet 42 of FIG. 2A).

The other influential component of atomization involves the influence of solvent evaporation. Over time, solvent will evaporate. For an individual atomized particle, the longer time-of-flight a particle has, the more solvent will be removed from that particle. Particles begin as large and high solvent content droplets (primary atomization region 14), transition to smaller droplets with lower solvent content (optimal droplet region 16), and lastly transition to even smaller and tacky polymer spheres (low-solvent content region 18). For this reason, particles in the secondary atomization region, and in particular, the optimal droplet zone 16 that contains small particles and sufficient solvent to spread on the coil surface, are utilized for the discrete spiral coating of the present invention.

In a preferred configuration, atomization is configured such that the droplets display low viscosity and low surface tension with respect to the coil material. The amount/concentration of solvent will generally control viscosity, which scales with surface tension. For example, for the same polymer-solvent system, more solvent would lower viscosity and surface tension, which produce better spreading and adhesion to substrate. Incidentally, the reservoir of the sprayer may also be heated to decrease viscosity, surface tension, and also make finer droplets because of faster solvent evaporation. Increasing the air-liquid flow ratio will also increase evaporation and make smaller droplets, but without thermal energy the viscosity and surface tension would increase. Since wetting is the final desired result, in a preferred embodiment the droplet in the optimized zone 16 is ideally configured to produce a wetting angle of less than 30 degrees on the coil substrate.

FIG. 2A through FIG. 2C illustrate the importance of droplet size control and coil stretching with respect to GDC 30. Spiral coating by atomized polymer deposition can be created using any GDC size, and thus the scale bar is provided for reference only.

In FIG. 2A, GDC 30 is not stretched (e.g. groove having spacing gᵣ). Sphere 40 represents a large, non-optimal droplet which will cover and occlude the helical gap or grooves of the GDC coil 30 and will not produce a discrete spiral coating. For purposes of this description, "occlusion" of the helical gap refers to a continuous or semi-continuous coating layer across the helical gap so as to close off the gap from one segment 32 to an adjacent segment, which may result in binding the coil 30 to restrict motion of the coil segments with respect to each other. Sphere 42 represents a small optimal droplet, in accordance with droplets disposed within optimal droplet zone 16 of FIG. 1, that are configured to more likely produce a discrete spiral coating on the outer surface 34 of the individual segments 32 of coil 30. Based on the resting gap/groove size gᵣ, the grooves of the GDC coil may still be occluded, even with the smaller droplet size 42.

In accordance with a preferred method of this presentation illustrated in FIG. 2B, the GDC coil 30 is stretched via application of tensile force F along the coil axis to increase the distance gₛ between adjacent coil segments 32 to create a linear target for deposition, as well as increase the gap between adjacent wire segments 32 to thereby increase coating efficiency for discrete spiral coating outer surface 34. Generally, the vast majority of the deposited coating is restricted to outer surface 34, leaving the inner surface 36 with a minimal amount of coating.

As shown in FIG. 2C, a mandrel 38 may optionally be inserted down the center of coil 30 to further mask internal surface 36 from deposition.

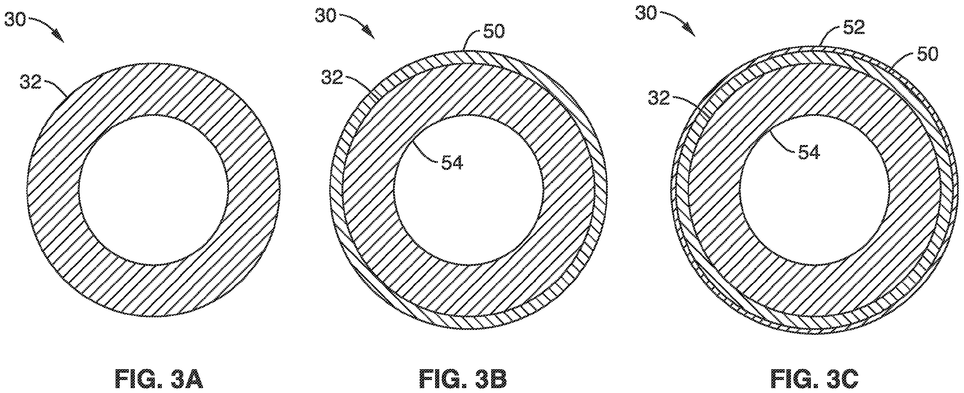

FIG. 3A through FIG. 3B illustrate cross-sectional views of coil 30 through various stages of a coating process of this presentation. FIG. 3A shows a view of the uncoated coil 30 showing only the metallic of coil segments 32. FIG. 3B illustrates the outside surface of segments 32 coated with bioactive polymer layer 50. It should be noted that inner surface 54 is substantially free of coating. FIG. 3B illustrates the outside surface of segments 32 coated with bioactive polymer layer 50 and lubricant layer 52. Again, inner surface 54 is substantially free of coating.

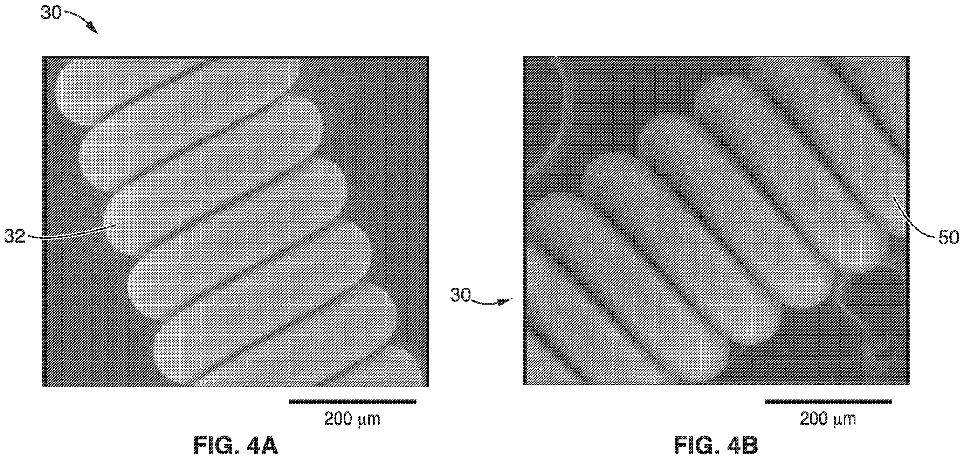

FIG. 4A shows a SEM image of an uncoated GDC 30. FIG. 4B shows a SEM image of the coated GDC 30 with bioactive polymer 50 in accordance with the processes of this presentation. As seen in FIG. 4B, the coating is smooth, homogenous, even, and covers the exterior of the metallic surface, leaving the helical grooves/gap substantially free from occlusion of the polymer, i.e. devoid of polymer.

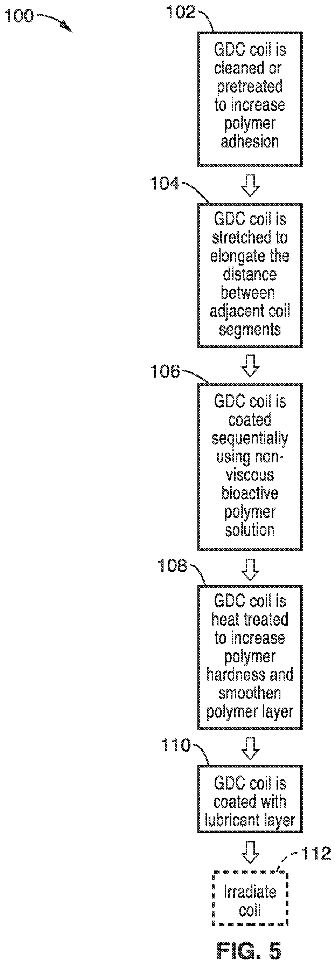

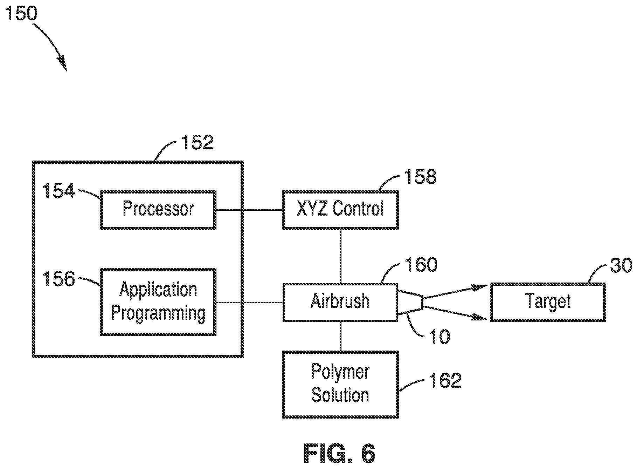

FIG. 5 is a flow diagram of a coating process 100 in accordance with this presentation. Method 100 may be used in conjunction with deposition system 150 shown in FIG. 6 for the coating of a target such as GDC 30.

First, at block 102, the coil 30 is optionally pretreated, e.g. with a cleansing solution, to increase polymer adhesion.

In a preferred embodiment, the coils 32 of GDC 30 are stretched elastically along the coil axis at step 104, as shown in FIG. 2B. Doing so creates a linear target, as well as increases the gap gₛ between adjacent coil segments 32.

Next, at step 106, the external surface 34 of coil 30 is discretely coated sequentially using a non-viscous bioactive polymer solution 162 (FIG. 6).

Parameters which influence discrete spiral coating include: solution viscosity, solvent evaporation rate, spray distance away from target (e.g. within optimal droplet zone 16), and rate of polymer deposition.

Factors which influence particle size are: solution surface tension, dispensing air pressure, solution flow rate, deposition rate, and atomization technique employed. By manipulating these variables, atomized polymer droplets 42 can be created small enough such that these particles are smaller than the gaps between adjacent wire segments 32.

In one embodiment, a 1% acidic 50:50 PLGA solution 162 in acetone (w/w) is created. In one embodiment, the following solution dispensing parameters may be used using conventional air-assisted atomization:
(a) Air Pressure: 15PSI to 25PSI,
(b) Solution flow rate per airbrush: 0.001g/s to 0.01g/s,
(c) Airbrush-to-target distance: 2cm to 5cm (e.g. distance D₁ to D₂ from nozzle exit 20),
(d) Translational velocity: 1 cm/s to 10cm/s.

Angular tilting (in x-y-z, and use of hydraulic airbrushes may also be considered. It is appreciated that the above parameters are variable, and may vary according to deposition technique, solution, coil size, etc.

The solution 162 is then loaded into one or more airbrushes 160 (e.g. an array of 3 airbrushes) attached to a robotic polymer atomizing robot (x-y-z control 158). The robot 158 is controlled by a computer 152 having a processor 154 and programming 156 executable on the processor to modulate solution flow rate, deposition rate (controlled by vertical velocity along the coil axis), and the number of layers. The robot 158 can be mechanically modified to control droplet time-of-flight by moving the airbrush 160 closer to or away from the target 30.

In one embodiment, ultrasonic atomization may also be used to achieve the discrete spiral coating of step 106 by modulating additional parameters specific to the ultrasonic atomization process: e.g. amplitude and frequency.

Using these parameters, polymer is imparted onto the GDC surface 34 in incremental layers. However, it is important to note that before a subsequent layer is deposited, the previous layer should be allowed to dry for a short period of time before the next layer is deposited.

Post processing steps may also be applied to the coil surface to improve final polymer characteristics which include: polymer smoothness, discrete coil coating, and polymer durability.

In one embodiment, drying the polymer surface can be achieved at step 108 with heated or unheated air, infrared heat, or reducing surrounding air pressure.

The number of polymer layers deposited will dictate the final thickness. Discrete spiral coating can be maintained up to 20µm in thickness by utilizing atomized polymer deposition. However, using thinner polymer layers is preferred to maintain coil flexibility and limit polymer fragmentation. A sacrificial gap layer may also be incorporated.

In one embodiment, after the coils have been coated, the coils 30 are then transferred to an oven set at 37° C and allowed to dry overnight. Coils 30 are then heat set above their glass transition temperature for a short period. The heat setting step 108 performs two primary functions. The first is to create a smooth surface due to stray particles by the atomization process. The second is to improve polymer hardness, by alignment of crystalline regions.

Additional post-processing steps may be utilized to increase other polymer coating properties. A protective/lubricant polymer layer 52 may be deposited at step 110 such that the PLGA layer is protected during handling and delivery. Protective or lubricant coatings 52 may be in liquid state or solid state, but also preserve coil flexibility during deployment. Exemplary lubricants include, but are not limited to: hypromellose, carboxymethyl cellulose, Eudragit S100, etc.

Final polymer characteristics may be improved at step 112 by radiation treatment to induce polymer cross-linking and provide sterilization. Gamma radiation may be used to increase polymer hardness and to address other handling and durability characteristics. Irradiation may also comprise electron beam, UV, or other sources available in the art.

The embodiments described in FIG. 1 through FIG. 6 above may also incorporate, or be combined with the concepts and embodiments described in prior PCT international patent application PCT/US2012/044049 filed on June 25, 2012 and published as PCT International Publication No. WO 2013/006298 A2 on January 10, 2013.

In one embodiment, an endovascular device comprises a metallic spiral coil 30 and a coating 50 on the coil wherein the coating is formed on the outer surface 34 of the spiral coil only such that the grooves of the coil remain uncoated and substantially free of the coating.

In some embodiments of the endovascular device, the metallic spiral coil 30 comprises platinum, tungsten, titanium, silver, stainless steel, zirconium, or an alloy thereof. In some embodiments, the metallic spiral coil 30 comprises Nitinol, polymers, or a biodegradable metal or alloy (e.g., magnesium or an alloy thereof).

In some embodiments of the endovascular device, optionally in combination with any or all of the above various embodiments, the coating 50 comprises a bioabsorbable polymer or a biodurable polymer. In some embodiments, the bioabsorbable polymer comprises a polyester polymer, e.g., polyglycolic acid (PGA), poly-L-lactic acid (PLLA), polycaprolactive, poly-L-lactide, polydioxanone, polycarbonates, polyanhydrides, polyglycolic acid/poly-L-lactic acid copolymers, and polyhydroxybutyrate/hydroxyvalerate copolymers, or combinations thereof.

In some embodiments, the biodurable polymer comprises polyacrylate, polymethacrylate, polyether, or a fluorinated polymer.

In some embodiments, the polymer can be polylactone, poly-alpha-hydroxy acids, poly(3-hydroxyalkanoates), polyglycols, polytyrosine carbonates, starch, gelatins, cellulose as well as blends and interpolymers containing these components. Examples of poly-alpha-hydroxy acids are polylactides, polyglycol acids, and their interpolymers.

In some embodiments, the polymer can be caprolactone/glycolide copolymer or calcium stearoyl lactylate. Calcium stearoyl lactylate degrades into stearic and lactic acids. The polymer can also be acidic polyesters, such as a mixture of PLGA and hydroxyacetic acid (about equivalent molar ratios), or polyester anhydrides such as glycolic acid, lactic acid, or sebacic acid polymers.

In some embodiments of the endovascular device, optionally in combination with any or all of the above various embodiments, the coating 50 further comprises a bioactive agent.

In some embodiments of the endovascular device, optionally in combination with any or all of the above various embodiments, the coating 50 comprises a drug matrix layer comprising a bioactive agent, an optional primer layer underneath the drug matrix layer, and an optional a top layer immediately over the drug matrix layer, and wherein the optional top layer provides a controlled release of the bioactive agent.

In some embodiments of the endovascular device, optionally in combination with any or all of the above various embodiments, the coating 50 further comprises a biobeneficial material that enhances biocompatibility of the coating. Such biobeneficial material can be any material capable of enhancing biocompatibility of the coating. Examples of such biobeneficial material can be, e.g., a material that comprises choline, e.g., phosphoryl choline.

The various above embodiments of the endovascular device can be any endovascular device. In some embodiments, the device is a detachable aneurysm coil. In some embodiments, the device is a bare platinum coil.

In another aspect of this presentation there is provided a method of forming a coating on an endovascular device. The device comprises a spiral coil body. The method comprises: forming a primary layer on the coil using a first solution comprising a primary layer material in a first solvent; removing the primary layer from the grooves of the spiral coil; forming a second layer on the outer surface of the spiral coil; using a second solution comprising a second layer material and a second solvent; drying the second layer; removing the primary layer from the grooves of the spiral coil; and drying the coating; wherein the primary layer material does not dissolve in the second solution and is not wet well by the second solution; and wherein the coating covers only the outer surface of the spiral coil.

Some embodiments of the method further comprise treating the coating with a solvent vapor to produce a smooth even coating.

In some embodiments of the method, optionally in combination with any or all of the above various embodiments, an additional lubricant layer may be deposited on top of the second polymer layer, which imparts additional advantages or desirable properties to the coating, e.g., to prevent damage to the polymer layer during storage, to confer polymer integrity during deployment, and/or to decrease friction during deployment. In some embodiments, the lubricant layer can also contain pro-inflammatory factors embedded within the lubricant layer, or possess inherent pro-inflammatory properties.

In general any combination of solvents can be used for the first or second solvent as long as they do not mix together, which is shown by high interfacial tensions and present disparate solubility parameters. In addition, the solvents must dissolve their respective polymers. The only first solvent we have tested was water. Second solvents that we have tested were: 1,2 Dichloroethane, 2-Phenoxyethanol, Acetone, Acetonitrile, Benzaldehyde, Benzonitrile, Benzyl alcohol, Chloroform, Dichloromethane, Dimethyl Adipate, Dimethyl sulfoxide, Dimethylformamide, Dioxane, Ethyl acetate, Hexafluoroisopropanol, Propylene carbonate. First and second solvents were chosen based on similar Hansen solubility parameters as the primary or secondary polymer, respectively. In some embodiments of the method, the first solvent is water, and the second solvent is chloroform.

In some embodiments of the method, optionally in combination with any or all of the above various embodiments, the primary layer material is dextran sulfate. Other materials for the primary layer material can be, e.g., polyethylene glycol, polyvinyl Alcohol, polyacrylic acid, polyvinylpyrrolidone, polyacrylamide, carboxymethyl cellulose, guar gum, hypromellose, glucose, polyvinyl sulfate, polyvinyl phosphonic acid, mowiol, hydroxyethyl cellulose, dextran, dextran sulfate, glycolide, pullan, starch, xylan, polyallylamie, polyepoxysuccinic acid, amylose, galactan, cellulose, gelatin, pectin, chitosan. The second layer material comprises a bioabsorbable polymer or a biodurable polymer. In some embodiments, the bioabsorbable polymer comprises a polyester, e.g., poly(lactic acid) (PLA), poly(lactic-co-glycolic acid) (PLGA), or a combination thereof. In some embodiments, the biodurable polymer comprises polyacrylate, polymethacrylate, polyether, or a fluorinated polymer. In some embodiments, the polymer can be polylactone, poly-alpha-hydroxy acids, poly(3-hydroxyalkanoates), polyglycols, polytyrosine carbonates, starch, gelatins, cellulose as well as blends and interpolymers containing these components. Examples of poly-alpha-hydroxy acids are polylactides, polyglycol acids, and their interpolymers. In some embodiments, the polymer can be caprolactone/glycolide copolymer or calcium stearoyl lactylate. Calcium stearoyl lactylate degrades into stearic and lactic acids.

In some embodiments of the method, optionally in combination with any or all of the above various embodiments, the second layer polymer comprises a material that generates a transient and mild inflammation so as to accelerate wound healing. Examples of such pro-inflammatory coating materials are acidic polyesters which are examples of pro-inflammatory coating materials that can accelerate healing. The polymer can also be acidic polyesters, such as a mixture of PLGA and hydroxyacetic acid (about equivalent molar ratios), or polyester anhydrides such as glycolic acid, lactic acid, or sebacic acid polymers. In some embodiments, where the second layer polymers are not inflammatory, the coating may contain fillers or particles that happen to cause transient and mild inflammation.

In the method of embodiments of this presentation, the various features of the spiral coil including the polymer, the coating, the layers of coating, and the bioactive agent are as described above or below.

In the method embodiments of this presentation, the various above embodiments of the endovascular device can be any endovascular device. In some embodiments, the device is a detachable aneurysm coil. In some embodiments, the device is a bare platinum coil.

In one embodiment, there is provided a method of forming a coating on a spiral coil. The method comprises pre-stretching and without pre-stretching techniques such as rolling, spraying, stamping, printing, etc. Other coating techniques include: direct dip coating, roll coating, spray coating, and geometric printing. All of these techniques - including the technique described above and below - may require the spiral coil to be stretched along the coil axis, prior to the coating methods, to expose the grooves such that the final coating is deposited exclusively on the coil surface.

Information on exemplary alternative coating techniques is provided below:

Direct dip coating - a spiral coil is immersed in a polymer solution (with appropriate solvent), withdrawn from the solution, and allowed to dry.

Roll coating - bioactive polymer is applied to a flat rubber stamping device. The bioactive polymer is applied to the spiral coil by touching the rubber stamp to an elongated spiral coil. The rubber stamp moves linearly along the coil, such that it rolls the coil. During this motion, the polymer releases from the rubber stamp, and is applied to the spiral coil.

Spray coating - a solution of bioactive polymer is prepared and is deposited onto the spiral coil surface by atomization. This process is similar to airbrushing or spray painting.

In some embodiments of the method of making a spiral coil, optionally in combination with any or all of the above various embodiments, the method comprises an optional step. This step will precede all coating steps. This step pertains to direct modification of the metal surface such that it increases the adhesion of the polymer to the metal surface. This technique can be achieved by increasing the surface area of the spiral coil, or increasing wetting of the polymer solution to the metal surface. Techniques to increase the surface area of the metal surface include: surface abrasion or acid etching. Techniques to increase the wetting of the polymer solution to the metal surface include plasma etching, plasma treatment, and surface cleaning.

There is described a method of treating or ameliorating a medical condition. (Said method does not form part of the invention). The method comprises implanting in a mammalian subject an endovascular device according to any of the various embodiments described above or below. In some embodiments, the medical condition is intracranial aneurysm rupture.

Embodiments of this presentation are advantageous in that they allow the modification of bare metallic coils (e.g., bare platinum coils) such that only selected surfaces along the spiral coil is coated with a polymer. This polymer coating can be bioactive, or may release a bioactive agent, or it may react with the local environment to provide bulking function. By leaving the grooves between each coil segment uncoated, the coating preserves the mechanical flexibility of the coil. Embodiments of this presentation can be applied to any currently available coil systems for the treatment of any medical condition that can be treated by an endovascular coil. An example of such medical conditions is brain aneurysms. Embodiments of this presentation will allow the coil material or system to have additional bioactive coating without impeding its mechanical property. Relatively large aneurysms will be treated more effectively so as to achieve less recanalization rate and improved treatment rate.

Additionally, the endovascular device provided herein is capable of generating a transient and mild inflammation condition at a site receiving the device or the surrounding area. A transient and mild inflammation condition can facilitate healing of a wound in a site receiving a device according to embodiments of this presentation. In some embodiments, acid polyesters can be coated onto a device disclosed herein to generate transient and mild inflammation at the site receiving the device.

### Definitions

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise. The practice of the present invention will employ, unless otherwise indicated, conventional methods of protein chemistry, biochemistry, and pharmacology, within the skill of the art. Such techniques are explained fully in the literature. See, e.g., T. E. Creighton, Proteins: Structures and Molecular Properties (W.H. Freeman and Company, 1993); A. L. Lehninger, Biochemistry (Worth Publishers, Inc., current addition); Remington's Pharmaceutical Sciences, 18th Edition (Easton, Pa.: Mack Publishing Company, 1990); each of which is incorporated herein by reference in its entirety.

The terms "effective amount" or "pharmaceutically effective amount" refer to a nontoxic but sufficient amount of the agent to provide the desired biological result. That result can be reduction and/or alleviation of the signs, symptoms, or causes of a disease, or any other desired alteration of a biological system. For example, an "effective amount" for therapeutic uses is the amount of the composition comprising a drug disclosed herein required to provide a clinically significant modulation in the symptoms associated with vascular permeability. An appropriate "effective amount" in any individual case may be determined by one of ordinary skill in the art using routine experimentation.

As used herein, the terms "treat" or "treatment" are used interchangeably and are meant to indicate a postponement of development of a disease associated with vascular permeability and/or a reduction in the severity of such symptoms that will or are expected to develop. The terms further include ameliorating existing symptoms, preventing additional symptoms, and ameliorating or preventing the underlying metabolic causes of symptoms.

The term "polymer" is defined as being inclusive of homopolymers, copolymers, and oligomers. The term "homopolymer" refers to a polymer derived from a single species of monomer. The term "copolymer" refers to a polymer derived from more than one species of monomer, including copolymers that may be obtained by copolymerization of two monomer species, those that may be obtained from three monomers species ("terpolymers"), those that may be obtained from four monomers species ("quaterpolymers"), etc. Some examples of polymers are bioabsorbable polymers and biodurable polymers. Further, as used herein, the term "polymer" includes any polymers that either directly, or indirectly by their degradation products will promote at least 25% increase in activities of neutrophils, macrophages, or other lymphocytes. Generally, such polymers do not include a polymer that tends to stick to itself when wet, as this would cause coil-coil friction during deployment and retrieval.

In some embodiments, the bioabsorbable polymer comprises a polyester, e.g., poly(lactic acid) (PLA), poly(lactic-co-glycolic acid) (PLGA), or a combination thereof or poly polyorthoesters. Bioabsorbable polymers can have acid, base, hydroxyl, or ester functional groups as side groups (pendant groups) or at one or both ends of the polymer backbone, which can also be referred to as acid-terminated, base-terminated, hydroxyl terminated, or ester terminated polymer. These polymers can be readily prepared according to established methodologies of polyester preparation. For example, acid terminated polyester can be readily prepared by using a diacid as the initiator in the preparation of the polyester. Likewise, amine-terminated (base-terminated), hydroxyl-terminated or ester-terminated polyester polymers can be readily prepared using a diamine, diol or an ester having a free hydroxyl group initiator in the preparation of the bioabsorbable polymer (e.g., PLA, PLGA, polyorthoester), respectively.

In some embodiments, the bioabsorbable polymer includes polymers that break down into acidic/basic monomers (e.g., PLA or polyorthoesters). The degradation products of these polymers can cause slightly inflammatory reactions.

In some embodiments, the biodurable polymer comprises polyacrylate, polymethacrylate, polyether, or a fluorinated polymer.

The term "poly(lactic acid-co-glycolic acid)" or "PLGA" refers to a copolymer formed by copolycondensation of lactic acid, HO--CH(CH₃)-COOH, and glycolic acid, HO--CH₂--COOH.

As used herein, the term "subject" encompasses mammals and non-mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. The term does not denote a particular age or gender.

By "substantially free" is meant that at least 80% or more (e.g., 90% or more, 95% or more, or 99%) area of the grooves of the spiral coil remain uncoated. Conversely, in some embodiments, by "substantially free" is meant that at least 80% or more (e.g., 90% or more, 95% or more, or 99%) area of the outer surface of the spiral coil remains uncoated.

By "does not dissolve" is meant the primary layer material has a solubility in the second solvent of lower than 1 g /100 cc.

By "is wet not well" is meant the primary layer and the second solution has a contact angle (θ) that is 90° or larger (θ ≥90°). The contact angle is the angle at which the liquid-vapor interface meets the solid-liquid interface. The contact angle is determined by the resultant between adhesive and cohesive forces. As the tendency of a drop to spread out over a flat, solid surface increases, the contact angle decreases. Thus, the contact angle provides an inverse measure of wettability. Adhesive forces between a liquid and solid cause a liquid drop to spread across the surface. Cohesive forces within the liquid cause the drop to ball up and avoid contact with the surface.

As used herein, the term "bioactive agent" can be any biologically active molecule. Any biologically active substance can be used as the source of biologically active molecules. Representative examples include laminin and growth factors such as IGF (insulin-like growth factors), TGF (transforming growth factors), FGB (fibroblast growth factors), including b-FGF (basic fibroblast growth factors), EGF (epidermal growth factors), VEGF (vascular endothelial growth factors), BMP (bone morphogenic proteins), PDGF (platelet-derived growth factors), or combinations thereof. These growth factors are well known and are commercially available.

The term "coil" can be any type of coil known in the art, such as, for example, a Guglielmi detachable coil (GDC). The coil can be coated with an absorbable polymeric material to improve long-term anatomic results in the endovascular treatment of intracranial aneurysms. The coil can further be coated to decrease friction to decrease the granulation tissue formation around the coils. In one embodiment, the coating comprises at least one biocompatible and bioabsorbable polymer and growth factors, and is used to accelerate histopathologic transformation of unorganized clots into fibrous connective tissue in aneurysms.

As used herein, the term "solvent vapor" generally refers to the vapor of a volatile solvent capable of dissolving a polymer for forming the second layer of a coating disclosed herein. The volatile solvent can be the same as or different from the solvent for the second solution for forming the second layer of coating. An example of the volatile solvent is acetone. Another example of the volatile solvent is ethyl acetate.

As used herein, the term "transient and mild inflammation" refers to an inflammatory condition limited to the site of tissue receiving a device disclosed herein and the surrounding area that would disappear or clear in a short period of time, e.g., hours or days. Such transient and mild inflammation is within the knowledge of a medical practitioner or researcher and can be measured by, e.g., a slight elevation of temperature (e.g., an increase of temperature of 0.5 F, 1 F, 1.5 F, or 2 F) at the site of tissue receiving the device and the surrounding area.

### Examples

The following examples are illustrative and not limiting.

In one example of forming a coating on a platinum/tungsten coil, the first step is performed by immersing the entire coil in an aqueous solution of dextran sulfate to form a primary layer, and then drawing the coil through a small aperture in a Teflon tape at controlled draw velocity to remove excess dextran sulfate. This first step confines the primary layer to the grooves of the coil. This dextran-coated coil is subsequently immersed and drawn from a polymer/chloroform solution (e.g., acid modified PLGA). The dextran sulfate can be replaced by any other polymer that does not dissolve in the second solution, and is not wet well by the second solution. After the PLGA is dried, the coil is immersed in water to remove the primary layer, and then dried. The coil is then flexed to remove any PLGA that has spanned over the grooves. Lastly the coil is exposed to acetone vapor to produce a smooth even coating which only covers the outer coil surface.

Although the description above contains many details, these should not be construed as limiting the scope of this presentation but as merely providing illustrations of some of the presently preferred embodiments of this presentation. All structural, chemical, and functional equivalents to the elements of the above-described preferred embodiment that are known to those of ordinary skill in the art are expressly incorporated herein by reference and are intended to be encompassed by the present claims. Moreover, it is not necessary for a device or method to address each and every problem sought to be solved by embodiments of this presentation, for it to be encompassed by the present claims. Furthermore, no element, component, or method step in the present disclosure is intended to be dedicated to the public regardless of whether the element, component, or method step is explicitly recited in the claims.

## Claims

1. A method of forming a coating on an endovascular spiral coil having a plurality of coil segments each separated by a helical gap, the method comprising:
providing a solution comprising a polymeric coating;
atomizing the polymeric coating a into a plurality of droplets at a set distance from the spiral coil;
adjusting droplet time-of-flight of the atomized polymeric coating according to the set distance from the spiral coil; and
coating an external surface of the spiral coil with the plurality of polymeric coating droplets;
wherein the droplets have a size smaller than the helical gap separating the plurality of coil segments such that internal surfaces with respect to the helical gap remain substantially free of occlusion by the polymeric coating.

2. A method as recited in claim 1, wherein the helical gap comprises a resting gap distance when disposed in an unloaded state, the method further comprising:
prior to coating the external surface, applying an axial tensile load on the spiral coil to expand the helical gap between the plurality of coil segments to an expanded distance greater than the resting gap distance.

3. A method as recited in claim 1:
wherein the solution comprises a solvent; and
wherein the set distance from the spiral coil is selected as a function of droplet size and solvent content.

4. A method as recited in claim 1, wherein the size of the plurality of droplets is adjusted by adjusting one or more of: solution viscosity, solvent evaporation rate, distance from the spiral coil, rate of polymer deposition; solution surface tension, deposition air pressure, solution flow rate, and deposition rate.

5. A method as recited in claim 1, further comprising:
depositing a layer of lubricant over the polymeric coating.

6. A method as recited in claim 1, further comprising:
heating the polymeric coating to harden the coating.

7. A method as recited in claim 1, further comprising:
irradiating the polymeric coating to induce polymer cross-linking within the coating.

8. A method as recited in claim 1, wherein the polymeric layer comprises a bioactive agent configured to promote wound healing.

9. A system for generating a coating on an endovascular spiral coil having a plurality of coil segments each separated by a helical gap, the system comprising:
a deposition nozzle for receiving a solution comprising a polymeric coating;
the deposition nozzle configured for atomizing the polymeric coating into a plurality of droplets at a set distance from the spiral coil;
the deposition nozzle further configured for coating an external surface of the spiral coil with the plurality of polymeric coating droplets;
an actuator coupled to the deposition nozzle for modifying the set distance from the nozzle to the spiral coil;
a processor coupled to the actuator and deposition nozzle; and
programming executable on the processor for:
adjusting position and deposition characteristics of the deposition nozzle to adjust the size of the plurality of droplets to have a size smaller than the helical gap separating the plurality of coil segments.

10. A system as recited in claim 9, wherein the programming is further configured for:
adjusting droplet time-of-flight of the atomized polymeric coating according to the set distance from the spiral coil.

11. A system as recited in claim 9, wherein the programming is further configured for:
adjusting the size of the plurality of droplets by controlling one or more of:
solution viscosity, solvent evaporation rate, distance from the spiral coil, rate of polymer deposition; solution surface tension, deposition air pressure, solution flow rate, and deposition rate.

## Patentansprüche

1. Verfahren zum Ausbilden einer Beschichtung auf einer endovaskulären Spirale mit mehreren Spiralensegmenten, die jeweils durch einen spiralförmigen Zwischenraum getrennt sind, wobei das Verfahren folgendes umfasst:
Bereitstellen einer Lösung, die eine polymeren Beschichtung umfasst;
Atomisieren der polymeren Beschichtung in eine Mehrzahl von Tröpfchen in einer festgelegten Entfernung von der Spirale;
Anpassen der Tröpfchenflugzeit der atomisierten polymeren Beschichtung gemäß der festgelegten Entfernung von der Spirale; und
Beschichten einer äußeren Oberfläche der Spirale mit der Mehrzahl polymerer Beschichtungströpfchen;
wobei die Tröpfchen eine Größe aufweisen, die kleiner ist als der spiralförmige Zwischenraum, der die Mehrzahl von Spiralensegmenten voneinander trennt, so dass innere Oberflächen in Bezug auf den spiralförmigen Zwischenraum im Wesentlichen frei von Okklusionen durch die polymere Beschichtung bleiben.

2. Verfahren nach Anspruch 1, wobei der spiralförmige Zwischenraum einen Ruhezwischenraumabstand aufweist, wenn er sich in einem ungeladenen Zustand befindet, wobei das Verfahren ferner folgendes umfasst:
vor dem Beschichten der äußeren Oberfläche, Anwenden einer axialen Zugbelastung auf die Spirale, um den spiralförmigen Zwischenraum zwischen der Mehrzahl von Spiralensegmenten auf einen erweiterten Abstand zu erweitern, der größer ist als der Ruhezwischenraumabstand.

3. Verfahren nach Anspruch 1:
wobei die Lösung ein Lösungsmittel umfasst; und
wobei die festgelegte Entfernung von der Spirale als eine Funktion der Tröpfchengröße und des Lösungsmittelinhalts ausgewählt wird.

4. Verfahren nach Anspruch 1, wobei die Größe der Mehrzahl von Tröpfchen angepasst wird durch Anpassen eines oder mehrerer der folgenden: Lösungsviskosität, Verdampfungsgeschwindigkeit des Lösungsmittels, Entfernung von der Spirale, Rate der Polymerabscheidung, Lösungsoberflächenspannung, Abscheidungsluftdruck, Lösungsströmungsgeschwindigkeit und Abscheidungsrate.

5. Verfahren nach Anspruch 1, wobei dieses ferner folgendes umfasst:
Abscheiden einer Schicht eines Gleitmittels über der polymeren Beschichtung.

6. Verfahren nach Anspruch 1, wobei dieses ferner folgendes umfasst:
Erhitzen der polymeren Beschichtung, um die Beschichtung zu härten.

7. Verfahren nach Anspruch 1, wobei dieses ferner folgendes umfasst:
Bestrahlen der polymeren Beschichtung, um eine Polymervernetzung in der Beschichtung zu induzieren.

8. Verfahren nach Anspruch 1, wobei die polymere Schicht eine bioaktive Substanz umfasst, die so gestaltet ist, um die Wundheilung zu fördern.

9. System zur Erzeugung einer Beschichtung auf einer endovaskulären Spirale mit mehreren Spiralensegmenten, die jeweils durch einen spiralförmigen Zwischenraum getrennt sind, wobei das System folgendes umfasst:
eine Abscheidungsdüse für den Empfang einer Lösung, die eine polymere Beschichtung umfasst;
wobei die Abscheidungsdüse so gestaltet ist, dass sie die polymere Beschichtung in eine Mehrzahl von Tröpfchen in einer festgelegten Entfernung von der Spirale atomisiert;
einen Aktuator, der mit der Abscheidungsdüse gekoppelt ist, um die festgelegte Entfernung von der Düse zu der Spirale zu modifizieren;
einen Prozessor, der mit dem Aktuator und der Abscheidungsdüse gekoppelt ist; und
eine Programmierung, die auf dem Prozessor zu folgendem Zweck ausgeführt werden kann:
Anpassen der Position und der Abscheidungseigenschaften der Abscheidungsdüse, um die Größe der Mehrzahl von Tröpfchen so anzupassen, dass sie eine Größe aufweisen, die kleiner ist als der spiralförmige Zwischenraum, der die Mehrzahl von Spiralensegmenten voneinander trennt.

10. System nach Anspruch 9, wobei die Programmierung ferner zu folgendem Zweck gestaltet ist:
Anpassen der Tröpfchenflugzeit der atomisierten polymeren Beschichtung gemäß der festgelegten Entfernung von der Spirale.

11. System nach Anspruch 9, wobei die Programmierung ferner zu folgendem Zweck gestaltet ist:
Anpassen der Größe der Mehrzahl von Tröpfchen durch Regelung eines oder mehrerer der folgenden: Lösungsviskosität, Verdampfungsgeschwindigkeit des Lösungsmittels, Entfernung von der Spirale, Rate der Polymerabscheidung, Lösungsoberflächenspannung, Abscheidungsluftdruck, Lösungsströmungsgeschwindigkeit und Abscheidungsrate.

## Revendications

1. Procédé de formation d'un revêtement sur un enroulement en spirale endovasculaire ayant une pluralité de segments d'enroulement séparés chacun par un espace hélicoïdal, le procédé comprenant les étapes consistant à :
fournir une solution comprenant un revêtement polymère ;
atomiser le revêtement polymère en une pluralité de gouttelettes à une distance définie de l'enroulement en spirale ;
ajuster le temps de parcours des gouttelettes du revêtement polymère atomisé en fonction de la distance réglée à partir de l'enroulement en spirale ; et
recouvrir une surface externe de l'enroulement en spirale avec la pluralité de gouttelettes de revêtement polymère ;
les gouttelettes ayant une taille plus petite que l'espace hélicoïdal séparant la pluralité de segments d'enroulement de sorte que les surfaces internes par rapport à l'espace hélicoïdal restent sensiblement exemptes d'occlusion par le revêtement polymère.

2. Procédé selon la revendication 1, l'espace hélicoïdal comprenant une distance d'espace de repos lorsqu'il est disposé dans un état non chargé, le procédé comprenant en outre l'étape consistant à :
avant de recouvrir la surface externe, appliquer une charge de traction axiale sur l'enroulement en spirale pour dilater l'espace hélicoïdal entre la pluralité de segments d'enroulement à une distance dilatée supérieure à la distance d'espace de repos.

3. Procédé selon la revendication 1 :
la solution comprenant un solvant ; et
la distance réglée à partir de l'enroulement en spirale étant choisie en fonction de la taille des gouttelettes et de la teneur en solvant.

4. Procédé selon la revendication 1, la taille de la pluralité de gouttelettes étant ajustée en ajustant un ou plusieurs des paramètres suivants : viscosité de la solution, vitesse d'évaporation du solvant, distance à partir de l'enroulement en spirale, vitesse de dépôt du polymère ; tension superficielle de la solution, pression d'air de dépôt, débit de la solution et vitesse de dépôt.

5. Procédé selon la revendication 1, comprenant en outre l'étape consistant à :
déposer une couche de lubrifiant sur le revêtement polymère.

6. Procédé selon la revendication 1, comprenant en outre l'étape consistant à :
chauffer le revêtement polymère pour durcir le revêtement.

7. Procédé selon la revendication 1, comprenant en outre l'étape consistant à :
irradier le revêtement polymère pour induire la réticulation du polymère à l'intérieur du revêtement.

8. Procédé selon la revendication 1, la couche polymère comprenant un agent bioactif conçu pour favoriser la cicatrisation des plaies.

9. Système pour générer un revêtement sur un enroulement en spirale endovasculaire ayant une pluralité de segments d'enroulement séparés chacun par un espace hélicoïdal, le système comprenant :
une buse de dépôt pour recevoir une solution comprenant un revêtement polymère ;
la buse de dépôt étant conçue pour atomiser le revêtement polymère en une pluralité de gouttelettes à une distance définie de l'enroulement en spirale ;
la buse de dépôt étant en outre conçue pour recouvrir une surface externe de l'enroulement en spirale avec la pluralité de gouttelettes de revêtement polymère ;
un actionneur accouplé à la buse de dépôt pour modifier la distance réglée entre la buse et l'enroulement en spirale ;
un processeur accouplé à l'actionneur et à la buse de dépôt ; et
un programme exécutable sur le processeur pour :
ajuster la position et les caractéristiques de dépôt de la buse de dépôt pour ajuster la taille de la pluralité de gouttelettes afin d'avoir une taille inférieure à l'espace hélicoïdal séparant la pluralité de segments d'enroulement.

10. Système selon la revendication 9, le programme étant en outre conçu pour :
ajuster le temps de parcours des gouttelettes du revêtement polymère atomisé en fonction de la distance réglée à partir de l'enroulement en spirale.

11. Système selon la revendication 9, le programme étant en outre conçu pour :
régler la taille de la pluralité de gouttelettes en commandant un ou plusieurs des paramètres suivants : viscosité de la solution, vitesse d'évaporation du solvant, distance par rapport à l'enroulement en spirale, vitesse de dépôt du polymère, tension superficielle de la solution, pression atmosphérique de dépôt, débit de la solution et vitesse de dépôt.
